# EUROPEAN PATENT APPLICATION

(11) **EP 4 717 153 A1**
(43) Date of publication of application: **01.04.2026**
(21) Application number: 24203435.3
(22) Date of filing: 30.09.2024
(51) Int. Cl.: A61B 5/00, A46B 15/00, A61B 5/11

(54) **ORAL CARE DEVICE CONTROL**

(71) Applicant: Koninklijke Philips N.V., 5656 AG Eindhoven (NL)
(72) Inventor: GOTTENBOS, Bart, Eindhoven (NL); SCHEFFERS, Lucas Petrus Henricus, Eindhoven (NL); BALTAZAR DOS SANTOS, Nuno Filipe, Eindhoven (NL)
(74) Representative: Philips Intellectual Property & Standards

(57) **Abstract**

Proposed concepts thus aim to provide schemes, solutions, concepts, designs, methods, and systems pertaining to aiding and/or improving image acquisition from an oral care device having an image capture device, the image of which may be influenced by the movement, positioning and/or applied pressure. In particular, embodiments aim to utilise data from one or more sensors to detect specific user behaviour during use of oral device. Such sensors may include pressure sensors, inertial measurement units (IMUs), or optical sensors. By analysing the data from such sensors, embodiments may, for example, effectively detect the occurrence of a predetermined user behaviour during use of oral device and, in turn, predict a preferred time to trigger image capture. In this way, improved images may be obtained.

## Description

### FIELD OF THE INVENTION

The present invention relates to the field of oral care devices, and in particular to the field of oral care devices that capture images of one or more oral features of a user.

### BACKGROUND OF THE INVENTION

It is known to provide oral care devices with the ability or functionality to capture digital impressions (e.g. images) of a feature or part of a user during use. For example, Intra-Oral Scanner (IOS) devices use projected light (e.g. laser or structured light) and an image sensor to capture images of the dentogingival tissues. These images can be processed to create a three-dimensional (3D) model of the scanned surface. Data provided by IOS can therefore be useful for oral care, including the detection of common dental pathologies such as caries, tooth discoloration, misalignment. Also, it may be advantageous to capture and compare repeated IOS images, to enable identification of changes in dentogingival tissues over time for example.

Traditional methods of capturing intraoral images often require manual operation of imaging devices, which can be challenging for both dental professionals and patients. The process typically involves carefully positioning the imaging device, maintaining stability, and manually triggering image capture at the appropriate moment.

One significant challenge in intraoral imaging is the need to capture high-quality images consistently while minimizing patient discomfort and reducing the time required for the imaging procedure. Factors such as motion blur, improper positioning, and inconsistent image quality can lead to the need for repeated imaging sessions, increasing patient discomfort and extending appointment durations. Additionally, the cognitive load on the operator to simultaneously manage device positioning, patient comfort, and image capture timing can be substantial.

Another problem in the field is the management of large volumes of image data generated during intraoral scanning procedures. Continuous image capture throughout a scan can result in excessive data storage and processing requirements, potentially slowing down workflows and increasing the complexity of image analysis tasks.

### SUMMARY OF THE INVENTION

The invention is defined by the claims.

According to an aspect of the invention, there is provided a method for controlling an oral care device having an image capture device adapted to capture images of one or more oral features of a user is provided. The method comprises: obtaining data describing at least one of: motion of the oral care device; a position of the oral care device; and pressure applied to the oral care device, during use of the oral care device; analysing the obtained data to detect an occurrence of a predetermined user behaviour during use of oral device; and generating, based on the detected occurrence of the predetermined user behaviour, a control signal for controlling image capture by the image capture device.

This may, for example, allow for automated and intelligent control of image capture during oral care routines, reducing cognitive load on the user and ensuring high-quality images are captured at optimal moments.

Proposed concepts thus aim to provide schemes, solutions, concepts, designs, methods, and systems pertaining to aiding and/or improving image acquisition from an oral care device having an image capture device, the image of which may be influenced by the movement, positioning and/or applied pressure. In particular, embodiments aim to utilise data from one or more sensors to detect specific user behaviour during use of oral device. Such sensors may include pressure sensors, inertial measurement units (IMUs), or optical sensors. By analysing the data from such sensors, embodiments may, for example, effectively detect the occurrence of a predetermined user behaviour during use of oral device and, in turn, predict a preferred time to trigger image capture. In this way, improved images may be obtained.

Embodiments may, for instance, provide an automated imaging trigger method/system for oral/dental cameras or intraoral scanners, addressing key challenges in the field of oral care imaging and diagnostics. The proposed approaches may leverage existing sensor technologies in oral care devices to intelligently control image capture, significantly improving the user experience and the quality of captured images.

A proposed concept of the invention is the utilization of sensor data, such as motion, position and pressure information, to detect specific user behaviours that indicate optimal moments for image capture. By analysing this data in real-time, a proposed method/system may automatically trigger the capture of high-quality images without requiring manual input from the user. Such automation may reduce the cognitive load on both dental professionals and subjects (i.e. patients) during imaging procedures.

A key advantage provided is the ability to selectively capture images at optimal moments, addressing issues of motion blur and inconsistent image quality that often plague traditional intraoral imaging methods. By only capturing images when the device is stable and properly positioned, the proposed method/system may help to ensure a higher percentage of usable, high-quality images, potentially reducing the need for repeated scans and improving overall efficiency.

Furthermore, embodiments may address the challenge of managing large volumes of image data. By implementing a dual-resolution approach - continuously capturing a low-resolution video stream while only triggering high-resolution image capture at specific moments - some embodiments may optimize data storage and processing requirements. Such an approach maintains situational awareness throughout the scanning process while focusing computational resources on the most valuable image data.

The flexibility of the proposed concept(s) allows for various implementations, including the use of different sensor types and user behaviour detection methods. Such adaptability helps to ensure that the method/system can be tailored to different clinical needs and integrated into a wide range of oral care devices, potentially revolutionizing the field of intraoral imaging and improving oral health outcomes for patients.

The predetermined user behaviour may comprise at least one of: applying the oral care device to an oral surface with a predetermined force; and pausing movement of the oral care device for a predetermined duration. By detecting specific user behaviours, the embodiments may more accurately determine when the oral care device is in an optimal position for image capture, improving the overall quality and relevance of captured images.

In some embodiments, analysing the obtained data may comprise: determining a movement speed of the oral care device; and detecting an occurrence of the movement speed meeting a predetermined speed requirement. Optionally, the predetermined speed requirement may require the movement speed to be less than a threshold speed value for a predetermined duration. Such analysis may enable the identification of moments of relative stability, ensuring that captured images are clear and free from motion blur.

In an embodiment, analysing the obtained data may comprise: detecting an occurrence of the pressure applied to the oral care device meeting a predetermined pressure requirement. Optionally, detecting an occurrence of the pressure applied to the oral care device meeting a predetermined pressure requirement may comprise comparing the pressure applied to the oral care device against a threshold pressure value. For example, the predetermined pressure requirement may require the pressure applied to the oral care device exceed a first threshold pressure value for a predetermined duration. In another example, the predetermined pressure requirement may require the pressure applied to the oral care device to not exceed a second threshold pressure value for a predetermined duration. By monitoring applied pressure, such embodiments may ensure that the oral care device is in proper contact with oral surfaces, leading to more consistent and accurate image capture.

Analysing the obtained data may comprise: detecting changes in pressure or motion patterns indicative of transitioning between different dental surfaces. This may enable an embodiment to automatically track the user's progress through the oral cavity, ensuring comprehensive coverage during imaging sessions.

The obtained data may comprise optical sensor data, and analysing the obtained data may comprise: implementing at least one of: edge detection and feature extraction on the optical sensor data to detect anatomical landmarks that indicate target positioning of the oral care device. By incorporating optical sensor data and advanced image processing techniques, such embodiments may more precisely identify optimal imaging positions based on oral anatomy.

In some embodiments, obtaining routine performance data may comprise sensing, with one or more sensors, at least one of: motion of the oral care device; a position of the oral care device; and pressure applied to the oral care device, wherein the one or more sensors comprise at least one of: a pressure sensor, an inertial measurement unit (IMU), or an optical sensor. The use of multiple sensor types may allow for more robust and accurate detection of user behaviour and device positioning.

The control signal may comprise a trigger for triggering image capture by the image capture device. Such direct triggering mechanism may help to ensure that images are captured at the exact moment when optimal conditions are detected.

In an embodiment, the image capture device may be configured to continuously capture a video stream at a first resolution, and the control signal may be configured to trigger the image capture device to capture an image at a second resolution that is higher than the first resolution. This dual-resolution approach may allow for continuous monitoring while conserving data storage and processing resources, only capturing high-resolution images when necessary.

Embodiments may further comprise: providing an alignment feature to assist in positioning the oral care device relative to an oral surface of the user. The inclusion of an alignment feature may improve user experience and helps ensure consistent and accurate device positioning during image capture.

According to another aspect of the invention, there is provided a computer program product for controlling an oral care device having an image capture device adapted to capture images of one or more oral features of a user is provided. The computer program product comprises a computer-readable storage medium having program instructions embodied therewith, the program instructions executable by a processor to cause the processor to perform a method comprising: obtaining data describing at least one of: motion of the oral care device; a position of the oral care device; and pressure applied to the oral care device, during use of the oral care device; analysing the obtained data to detect an occurrence of a predetermined user behaviour during use of oral device; and generating, based on the detected occurrence of the predetermined user behaviour, a control signal for controlling image capture by the image capture device.

Such a computer program product may enable the implementation of the intelligent image capture control method in software, allowing for easy updates and customization of the system's behaviour.

According to yet another aspect of the invention, there is provided a system for controlling an oral care device having an image capture device adapted to capture images of one or more oral features of a user is provided. The system comprises: an interface configured to obtain data describing at least one of: motion of the oral care device; a position of the oral care device, and pressure applied to the oral care device, during use of the oral care device; a processor arrangement configured to analyse the obtained data to detect an occurrence of a predetermined user behaviour during use of oral device; and a controller configured to generate, based on the detected occurrence of the predetermined user behaviour, a control signal for controlling image capture by the image capture device.

Such a system can provide a hardware implementation of the proposed image capture control method, allowing for integration into various oral care device designs.

The system may further comprise one or more sensors configured to sense at least one of: motion of the oral health care device; a position of the oral care device; and pressure applied to the oral health care device. The inclusion of integrated sensors allows for direct measurement of device motion and applied pressure, improving the accuracy of user behaviour detection.

The one or more sensors may comprise at least one of: a pressure sensor, an inertial measurement unit (IMU), or an optical sensor. Such use of multiple sensor types provides redundancy and allows for more sophisticated analysis of user behaviour and device positioning.

The system may further comprise: an alignment feature to assist in positioning the oral care device relative to an oral surface of the user. The inclusion of a physical alignment feature enhances the system's ability to guide users in proper device positioning, further improving the quality and consistency of captured images.

Embodiments may be of particular relevance to oral care devices having an image capture device (e.g. digital camera), such as Intra-Oral Scanners (IOSs), dental/oral cameras and electric toothbrushes for example. Thus, according to another aspect of the invention, there may be provided an oral care device having: an image capture device adapted to capture images of one or more oral features of a user; and a system for controlling the oral care device according to a proposed embodiment.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a flowchart of a method for controlling an oral care device according to an embodiment of the invention;
Fig. 2 illustrates a block diagram of a system for controlling an oral care device, in accordance with an embodiment of the invention;
Fig. 3 shows an orthogonal side view of an oral care device incorporating the system of Fig. 2, according to an embodiment; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.
It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to assessing an oral health care routine of a user. According to proposed concepts, a number of possible solutions may be implemented separately or jointly. That is, although these possible solutions may be described below separately, two or more of these possible solutions may be implemented in one combination or another.

The invention proposes concepts for aiding and/or improving image acquisition from an oral care device having an image capture device. In particular, embodiments may provide a system, device and/or method which controls image capture by the image capture device based on a detected occurrence of a predetermined user behaviour. In particular, embodiments propose to utilise data from one or more sensors to detect specific user behaviour during use of the oral care device. Such sensors may include pressure sensors, inertial measurement units (IMUs), or optical sensors. Through such control of image capture, improved images (e.g. images exhibiting less blur and/or distortion) may be obtained.

In other words, embodiments propose to control the timing of image capture based on the detecting certain user behaviour that is indicative of an optimal/preferred time for image capture, thus facilitating the capture of images of improved quality.

Embodiments provide a system and/or method for controlling an oral care device equipped with an image capture device. The system/method is configured to intelligently automate the process of capturing images of various oral features of a user during an oral care routine (such as measuring/imaging procedure, for example). The system/method leverages data obtained from one or more sensors that monitor the motion, position, and/or applied pressure of the oral care device during use. This data is analysed to detect specific user behaviours that may be indicative of optimal moments for image capture. Responsive to detection of such behaviours, a control signal is generated for controlling (e.g. triggering) the image capture device, thereby automating the image capture process and reducing the cognitive load on the user. Embodiments may therefore help to ensure that high-quality images are captured at the appropriate moments, thereby optimizing data storage and processing requirements.

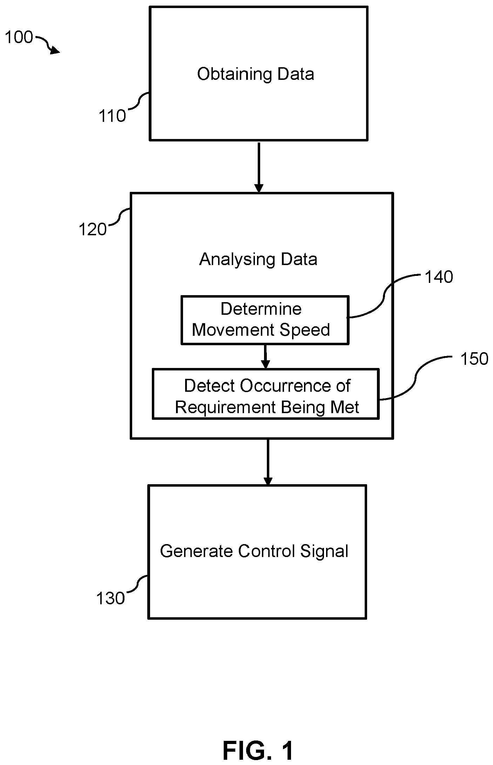

Referring now to Fig. 1, there is depicted a flow diagram of a method 100 for assessing an oral health care routine performed using an oral health care device according to a proposed embodiment.

FIG. 1 illustrates a flowchart of a method 100 for controlling an oral care device with an image capture device. The flowchart depicts a sequential process where each step leads to the next, showing how the method 100 progresses from data collection to analysis and control signal generation. The method 100 utilizes obtained data to identify behaviours that may be indicative of optimal moments for image capture.

The method 100 begins with step 110, which involves obtaining data. This data describes motion, position, and/or pressure applied to the oral care device during use (e.g. during the performance of an oral health care routine).

Motion data may be obtained through various sensors that are integrated into (or coupled to) the oral care device, such as an inertial measurement unit (IMU) or an optical sensor. The IMU may be configured to track the motion and position of the oral care device. For instance the IMU may be configured to measure the acceleration and angular velocity of the oral care device, providing detailed information about the device's movement patterns and/or position during performance of the oral health care routine.

Similarly, pressure data may be obtained through one or more pressure sensors integrated into the oral care device. The pressure sensors may be configured to measure the force exerted by the user on the oral care device, providing information about user's interaction with the device and/or the contact between the device and the teeth during performance of the oral health care routine. For example, changes in pressure may indicate transitions between different dental surfaces, such as transitions between teeth surfaces and interproximal spaces (which can be used to identify a relative position of oral care device during performance of the oral health care routine, for example).

Pressure data (i.e. routine performance data) may be used in conjunction with the motion data to enhance the accuracy of the behaviour identification.

Further, an optical sensor may be utilized to detect the proximity of the oral care device to the oral surface of the user, contributing to the determination of optimal image capture.

The method 100 then proceeds to step 120, which involves analysing the obtained data to detect an occurrence of a predetermined user behaviour during use of oral device.

By way of example, the predetermined user behaviour in this embodiment involves the user pausing the movement of the oral care device for a certain duration. This pause in movement may be detected using date from the IMU. Detection that the oral care device is stationary or moving slowly for a predefined duration, may be interpreted as an indication that the user is holding the device still, which may be an optimal moment for image capture.

Within step 120, there are two sub-steps: step 140 for determining movement speed, and step 150 for detecting the occurrence of a requirement being met. Step 140 involves calculating the speed at which the oral care device is moving, while step 150 involves checking if this speed meets a predetermined requirement, such as being below a certain threshold for a specific duration.

In this example, the embodiment employs a predefined speed requirement that requires the movement speed of the oral care device to be less than a threshold speed value for a predetermined duration. This requirement is based on the assumption that a slower movement speed or a pause in movement may indicate an optimal moment for image capture. When the movement speed of the device is detected to be less than the threshold speed value for the predetermined duration, a control signal may be generated to trigger the image capture device to capture an image.

The analysis step 120 may implement machine learning algorithms or other data analysis techniques to identify user behaviours that may be indicative of optimal moments for image capture. These algorithms may be trained on datasets captured under various conditions, and they may be configured to identify patterns or features in motion, position and/or pressure data that correlate with optimal imaging conditions. Such use of machine learning algorithms may enhance the accuracy and efficiency of the image capture control process, thereby improving the overall performance of the oral care device.

Following the analysis in step 120, the method 100 moves to step 130, which involves generating a control signal. This control signal is based on the detected occurrence of the predetermined user behaviour identified in the previous step. The control signal may be used to trigger image capture by the image capture device of the oral care device. For instance, the control signal may serve as a trigger for the image capture device, instructing it to capture an image at the right moment. The trigger may be configured to activate the image capture device instantly, ensuring that the image is captured at the moment when the predetermined user behaviour is detected.

The flowchart demonstrates a sequential process of data acquisition, analysis, and signal generation, with the analysis step 120 incorporating specific checks on movement speed and requirement fulfilment. This method 100 therefore allows for automated control of image capture based on user behaviour during the use of the oral care device. Such automated triggering of image capture may reduce the cognitive load on the user and ensure that high-quality images are captured at optimal moments.

Variations to the steps detailed above for the embodiment of Fig. 1 may be employed.

For example, some embodiments may analyse the obtained data to detect an occurrence of the pressure applied to the oral care device meeting a predetermined pressure requirement. This analysis may be facilitated by a pressure sensor integrated into the oral care device. The pressure sensor may be configured to measure the force exerted by the user on the device, and analysis of the pressure data may identify when the measured force exceeds a first pressure threshold value. This signal may be interpreted as an indication that the user is pressing the device against an oral surface, which may be an optimal moment for image capture. Alternatively, or additionally, in some embodiments, analysis of the pressure data may identify when the measured force does not exceed a second pressure threshold value. This signal may be interpreted as an indication that the user is not pressing the device against an oral surface with excessive force, which may otherwise distort or negatively impact image capture.

Other embodiments may analyse the obtained data to detect changes in pressure or motion patterns indicative of transitioning between different dental surfaces. This analysis may be facilitated by a combination of pressure sensors and inertial measurement units (IMUs) integrated into the oral care device. By analysing the data obtained from these sensors, the system may be able to detect when the user is transitioning from one dental surface to another, such as from a tooth surface to an interdental space. This detection may be based on changes in the measured force or motion patterns, which may indicate a change in the contact surface of the device. When such a transition is detected, a control signal may be generated to trigger the image capture device to capture an image. This may ensure that images are captured at key moments during the oral care routine, thereby enhancing the coverage and quality of the captured images.

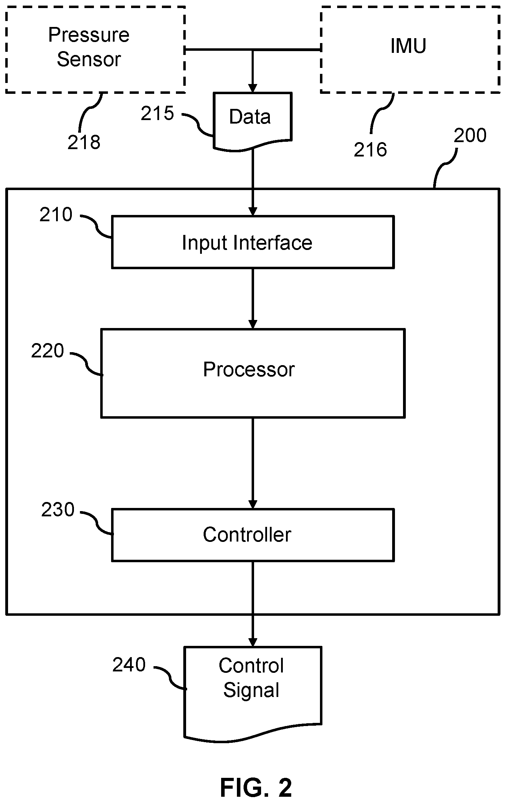

Referring now to Fig. 2, there is depicted a block diagram of a system 200 for controlling an oral care device with an image capture device. The image capture device is configured to capture images of one or more oral features of the user. Further, the image capture device is controllable by a control signal. The system 200 is designed to process sensor data from the oral care device, analyse the data, and generate a control signal for controlling image capture by the image capture device.

The system 200 has an input interface 210 in communication with a pressure sensor 218 and an Inertial Measurement Unit (IMU) 216 so as to receive routine data 215 from the sensors. The pressure sensor 218 is configured to measure the force exerted by the user on the oral care device, providing information about the contact between the device and the teeth. The IMU 216 is configured to measure the acceleration and angular velocity of the oral care device, providing detailed information about the device's movement patterns and/or position(s). The input interface 210 is thus configured to receive data describing the motion and position of the oral care device and the pressure applied to the device during the performance of the oral health care routine. The interface may also be configured to receive data from an optical sensor, for example.

The input interface 210 forwards the received data 215 from the sensors 216, 218 to a processor 220 of the system 200. The processor analyses the data 215 to detect an occurrence of a predetermined user behavior during the use of the oral care device.

In particular, the processor 220 is configured to analyse the data to identify a specific pattern of motion or pressure application that indicates a preferable moment for image capture. In this example, the user behaviour involves applying a certain amount of pressure to the oral care device, moving the device in a certain way, or pausing the device for a certain duration. The processor arrangement may be configured to detect these behaviours by analysing the data obtained from the sensors and comparing it to predefined criteria or thresholds. For example, the processor 220 is configured to detect when the measured force exceeds a predefined threshold. This may be interpreted as an indication that the user is pressing the device against an oral surface, such as a tooth or an interdental space, which may be an optimal moment for image capture.

The processor 220 is connected to the controller 230, which generates a control signal 240 based on the processed data. The control signal 240 is output from the system 200 for controlling the oral care device.

Here, the control signal is configured to trigger image capture by the image capture device of the oral care device. The control signal thus serves as a trigger for the image capture device, instructing it to capture an image at a preferred moment. The trigger may be configured to activate the image capture device instantly, ensuring that the image is captured at (substantially) the same moment when the predetermined user behaviour is detected, thus ensuring the capture of high-quality images. In other embodiments, however, the trigger may be configured to activate the image capture device at a specific time after detection of the user behaviour (e.g. at a predicted/calculated time offset).

The embodiment of Fig. 2 does not include the sensors. That is, the system of Fig. 2 is configured to leverage data from pre-existing sensors provided in/with the oral care device.

In alternative embodiments, the system may include one or more sensors configured to generate data. These sensors may be integrated into the oral care device and may include, for example, a pressure sensor, an inertial measurement unit (IMU), and/or an optical sensor. An optical sensor may, for example, be configured to detect the proximity of the oral care device to the oral surface of the user. More particularly, such an optical sensor may be configured to detect light reflected from the teeth, providing information about the shape and spacing of the teeth. This optical data may provide information about the shape and spacing of the teeth. For instance, changes in light reflection may indicate transitions between different dental surfaces, such as transitions between teeth surfaces and interproximal spaces. These transitions may be used as indicators of the presence of a dental feature, such as an interproximal space between teeth.

In some cases, a simple optical sensor similar to the optical component in a computer mouse may be used to detect when the oral care device is in a specific location. Additionally, or alternatively, an optical sensor may be configured to detect the motion of the device relative to the oral surface, and may be analysed to identify when the oral care device is stationary or moving slowly. This may be used as an indication that the user is pausing the device, which may be interpreted as a cue for image capture.

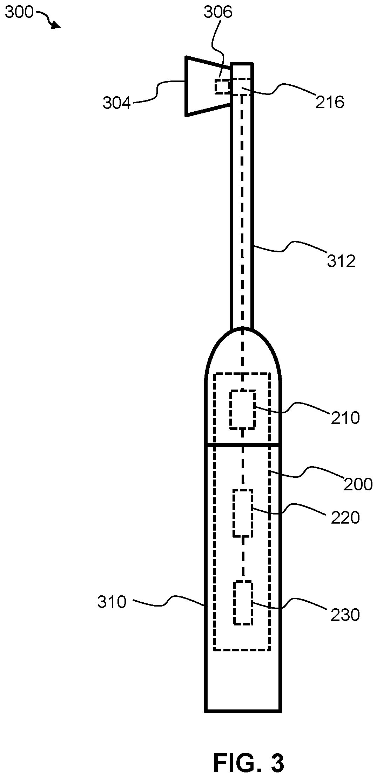

Fig. 3 illustrates a side view of an oral care device 300 incorporating the system 200 of Fig. 2. The oral care device 300 is an intraoral scanner which comprises a handle section 310 atop which is an elongated arm 312.

At the distal end of the arm is a conical section 304 which houses an image capture device 306. Also provided at the distal end of the arm 312, adjacent the optical sensor 306, is an IMU 216. The IMU 216 is configured to measure the acceleration and angular velocity of the oral care device, providing detailed information about the device's movement patterns.

In this example, the image capture device 306 itself may serve as a user behaviour sensor. The image capture device 306 may be configured to capture a continuous stream of images or video frames, which may be analysed to detect changes in the scene or the position of the device. For example, data from the image capture device 306 may be used to detect when the user is pausing the device or moving it to a typical imaging location, such as an interdental space or a specific tooth surface. This detection may be based on the recognition of certain visual features or patterns in the captured images, such as the shape of the teeth or the color of the oral tissues.

The system 200 is integrated within the handle section 310. The input interface 210 of the system is positioned near the top of the handle section 310, to receive data from the sensors 216, 306. Below the input interface 210 is the processor 220, which is responsible for analysing the data. The controller 230 of the system is situated at the bottom of the handle 300.

Connections link the sensors 216, 306 to the input interface 210, the input interface 210 to the processor 220, and the processor 220 to the controller 230. The connections thus facilitate the flow of data and signals within the system 200.

The arrangement of components within the oral care device 300 allows for efficient data processing and control of the image capture device 306 according to the proposed concept(s). The input interface 210 receives data from the sensors 306 and 216, the processor 220 analyses this data, and the controller 230 generate appropriate feedback or control signals based on the result(s) of the analysis.

The oral care device 300 integrates imaging capabilities with motion and position sensing. The scanner head 304 allows for intraoral imaging, while the IMU 216 can detect device motion and orientation. The internal components in the handle 312 process sensor data and control image capture, enabling automated imaging based on detected user behaviours during oral care routines.

Modifications to the oral care device 300 of Fig. 3 may be implemented.

For example, in some embodiments, the oral care device may also include an alignment feature to assist in positioning the oral care device relative to an oral surface of the user. The alignment feature may be designed to guide the user in positioning the oral care device at an optimal distance and angle relative to an oral surface of the user for image capture. The alignment feature may, for example, be shaped (e.g. wedge-shaped) to fit into the interdental spaces, thereby ensuring that the oral care device is positioned correctly for capturing images of the interdental spaces. This alignment element may also be equipped with contact sensors on both sides, which may be configured to detect when the alignment element is in contact with two opposing teeth. When the contact sensors detect such contact, they may generate a signal indicating that the alignment element is positioned in an interdental space (i.e. the data indicating the position of the oral care device). The alignment feature may also provide a tactile feedback to the user, indicating that the oral care device is positioned correctly for image capture.

Accordingly, in some embodiments, data from contact sensors associated with an alignment element may provide data that can be used to identify a position of the oral care device, as the positioning of the alignment element in an interdental space may indicate an optimal location for capturing images of the interdental spaces or the adjacent teeth.

Further, in some embodiments, one or more of the employed sensors may be integrated into different parts of the oral care device. For example, a pressure sensor may be integrated into a handle of the device, while an IMU and/or an optical sensor may be integrated into a brush head or scanning module of the device. Such a configuration may allow for more accurate and comprehensive sensing of the oral health care routine.

In some embodiments, the image capture device of the oral care device may be configured to continuously capture a video stream at a first resolution. This continuous capture may allow the system to monitor the oral cavity in real-time, providing a constant stream of visual data for analysis. The first resolution may be selected to balance the need for image detail with the constraints of data storage and processing power. In some cases, the first resolution may be a lower resolution that allows for efficient data handling while still providing sufficient detail for basic image analysis and user behavior detection.

The system may then generate a control signal that triggers the image capture device to capture an image at a second resolution that is higher than the first resolution. This trigger may be generated in response to the detection of a predetermined user behavior, such as the user applying a certain amount of pressure to the oral care device or pausing the movement of the device for a certain duration. The second resolution may be selected to provide a higher level of detail for the captured image, allowing for more accurate and detailed analysis of the oral features. The transition from the first resolution to the second resolution may be seamless, ensuring that the user experience is not disrupted.

In some implementations, the system may implement a local buffering system that temporarily stores the high-resolution images on the device. This local buffering system may serve as a temporary storage space for the high-resolution images before they are transmitted to an external storage location or processed by the system. The local buffering system may be designed to handle the larger data size of the high-resolution images, ensuring that the system's performance is not compromised by the increased data load.

Also, in some embodiments, the system may utilize edge computing techniques to perform initial image processing on the device itself. These edge computing techniques may involve processing the captured images at the edge of the network, close to the source of the data. This may reduce the amount of data that needs to be transmitted over the network, thereby reducing bandwidth requirements and improving system performance. The initial image processing may include tasks such as image enhancement, feature extraction, or user behavior detection, which may be performed on the device before the data is transmitted for further processing or storage.

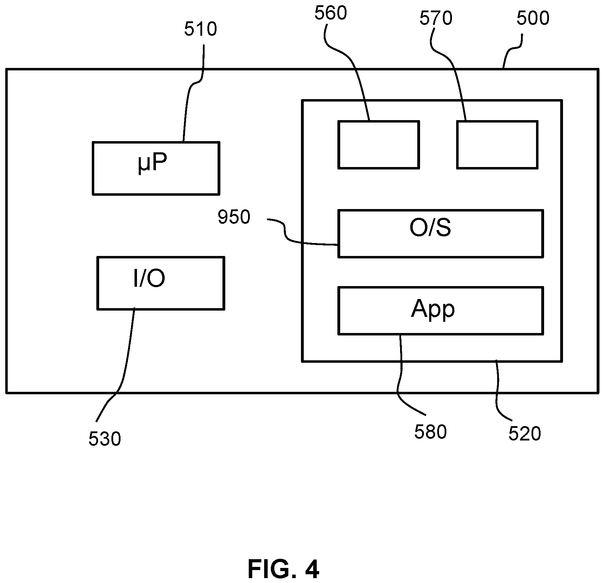

Fig. 4 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The embodiments of the Figures may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for controlling an oral care device (300) having an image capture device (306) adapted to capture images of one or more oral features of a user, the method comprising:
obtaining (110) data describing at least one of: motion of the oral care device; a position of the oral care device; and pressure applied to the oral care device, during use of the oral care device;
analysing (120) the obtained data to detect an occurrence of a predetermined user behaviour during use of oral device; and
generating (130), based on the detected occurrence of the predetermined user behaviour, a control signal (240) for controlling image capture by the image capture device.

2. The method of claim 1, wherein the predetermined user behaviour comprises at least one of:
applying the oral care device to an oral surface with a predetermined force; and
pausing movement of the oral care device for a predetermined duration.

3. The method of claim 1 or 2, wherein analysing the obtained data comprises:
determining (140) a movement speed of the oral care device; and
detecting (150) an occurrence of the movement speed meeting a predetermined speed requirement,
and optionally wherein the predetermined speed requirement requires the movement speed to be less than a threshold speed value for a predetermined duration.

4. The method of any of claims 1 to 3, wherein analysing the obtained data comprises:
detecting an occurrence of the pressure applied to the oral care device meeting a predetermined pressure requirement,
and optionally wherein detecting an occurrence of the pressure applied to the oral care device meeting a predetermined pressure requirement comprises
comparing the pressure applied to the oral care device against a threshold pressure value.

5. The method of any of claims 1 to 4, wherein analysing the obtained data comprises:
detecting changes in pressure or motion patterns indicative of transitioning between different dental surfaces.

6. The method of any of claims 1 to 5, wherein the obtained data comprises optical sensor data,
and wherein analysing the obtained data comprises:
implementing at least one of: edge detection; and feature extraction; on the optical sensor data to detect anatomical landmarks that indicate target positioning of the oral care device.

7. The method of any of claims 1 to 6, wherein obtaining routine performance data comprises sensing, with one or more sensors, at least one of: motion of the oral care device; a position of the oral care device; and pressure applied to the oral care device, and wherein the one or more sensors comprise at least one of: a pressure sensor (218), an inertial measurement unit, IMU 216; and an optical sensor.

8. The method of any of claims 1 to 7, wherein the control signal (240) comprises a trigger for triggering image capture by the image capture device.

9. The method of any of claims 1 to 8, wherein the image capture device is configured to continuously capture a video stream at a first resolution, and wherein the control signal is configured to trigger the image capture device to capture an image at a second resolution that is higher than the first resolution.

10. The method of any of claims 1 to 9, further comprising:
providing an alignment feature to assist in positioning the oral care device relative to an oral surface of the user.

11. A computer program product for controlling an oral care device (300) having an image capture device (306) adapted to capture images of one or more oral features of a user, the computer program product comprising a computer-readable storage medium having program instructions embodied therewith, the program instructions executable by a processor to cause the processor to perform a method comprising:
obtaining (110) data (215) describing at least one of: motion of the oral care device; a position of the oral care device; and pressure applied to the oral care device, during use of the oral care device;
analysing (120) the obtained data to detect an occurrence of a predetermined user behaviour during use of oral device; and
generating (130), based on the detected occurrence of the predetermined user behaviour, a control signal (240) for controlling image capture by the image capture device.

12. A system (200) for controlling an oral care device having an image capture device (306) adapted to capture images of one or more oral features of a user, the system comprising:
an interface (210) configured to obtain data describing at least one of: motion of the oral care device; a position of the oral care device; and pressure applied to the oral care device, during use of the oral care device;
a processor (220) arrangement configured to analyse the obtained data to detect an occurrence of a predetermined user behaviour during use of oral device; and
a controller (230) configured to generate, based on the detected occurrence of the predetermined user behaviour, a control signal for controlling image capture by the image capture device.

13. The system of claim 12 further comprising one or more sensors (216) configured to sense at least one of: motion of the oral health care device; a position of the oral care device; and pressure applied to the oral health care device, and optionally wherein the one or more sensors comprise at least one of: a pressure sensor, an inertial measurement unit (IMU), or an optical sensor.

14. The system of any of claims 12 to 13, further comprising:
an alignment feature to assist in positioning the oral care device relative to an oral surface of the user.

15. An oral care device (300) comprising: an image capture device (306) adapted to capture images of one or more oral features of a user; and a system (200) according to any of claims 12 to 14 for controlling the oral care device, and optionally wherein the oral device comprises an Intra-Oral Scanner.
